# EUROPEAN PATENT APPLICATION

(11) **EP 0 535 376 A1**
(43) Date of publication of application: **07.04.1993**
(21) Application number: 92114724.5
(22) Date of filing: 28.08.1992
(51) Int. Cl.: C12Q 1/68, G01N 33/538

(54) **Analytical method of polymer**

(30) Priority: 30.08.1991 JP 300002/91
(71) Applicant: IMMUNOBION LTD., Sapporo-shi, Hokkaido (JP)
(72) Inventor: Miwa, Shigeru, Sapporo-shi, Hokkaido (JP)
(74) Representative: Hansen, Bernd, Dr. Dipl.-Chem.

(57) **Abstract**

By contacting to a sample containing an analytical subject polymer and a low molecular substance an insoluble carrier preliminarily bound with an identical polymer to or a different polymer from the analytical subject polymer to saturation, the low molecular substance is adsorbed onto the insoluble carrier with no adsorption of the analytical subject polymer, whereby the analytical subject polymer can be separated from the low molecular substance. By contacting a mixture containing an analytical subject polymer and a low molecular substance to an insoluble polymer preliminarily bound with an identical polymer to or a different polymer from the analytical subject polymer to saturation, and measuring the amount of the low molecular substance non-adsorbent to the insoluble carrier, the amount of the low molecular substance specifically bound to the analytical subject polymer can be assayed, whereby it is determined whether or not the low molecular substance is specifically bound to the analytical subject polymer.

## Description

### BACKGROUND OF THE INVENTION

The present invention relates to fields of molecular genetics, biochemistry and immunochemistry. More specifically, the present invention relates to the separation of an analytical subject via an insoluble carrier bonded with a polymer, and also relates to the analytical method thereof.

Generally, hybridization is employed in order to examine the homology of nucleotide sequences of nucleic acids and the like. Because hybridisation is based on the formation of complementary base pairs, it is possible to detect the presence of a point mutation, insertion or deletion of DNA under an appropriate condition, which is very important for diagnosis of genetic diseases. The method for detecting the formation of a nucleic acid hybrid includes the observation under an electron microscope, a method using a solid support, electrophoresis, filtration method and the like.

In order to quantitatively assay an antigen-antibody reaction, a great number of methods have been developed, for example, immunodiffusion, laser nephrometry, and especially a labeled immunoassay at a high sensitivity.

In order to localize a homologous sequence or a different sequence on DNA, the observation under an electron microscope following a method for forming hetero-duplex DNA is not common because it requires a specific technique for sample preparation and the like, and it is extremely difficult to detect a point mutation.

A method comprising fixing a target nucleic acid on a solid support such as nitrocellulose membrane and nylon membrane and hybridising the nucleic acid with an appropriate labeled probe has problems in that it requires to continue the reaction for a prolonged period of time because the reaction progresses between a solid phase and a liquid phase, and in that unreacted probes should necessarily be removed with care so as to remove non-specific binding, and hence, the method demands tough efforts and a long period of time.

A nucleic acid-labeled probe hybrid and an unreacted labeled probe can readily be separated from each other, by electrophoresis having the function as a molecular sieve and gel filtration (described in Japanese Patent Laid-open No.63-2456985), but these methods nonetheless require a long time so that the screening of a great number of samples is difficult. Thus, the automatic processing of such methods is hard.

For an antigen-antibody reaction, immunodiffusion and laser nephrometry have limitation in terms of sensitivity and convenience to measure complex precipitates.

According to a labeled immunoassay at a high sensitivity, red blood cells, radioactive isotopes, enzymes, fluorescent materials or metals are employed, and a complex composed of a labeled antigen (or antibody) bonded to an antibody (or antigen) should be separated from an unbound labeled antigen (or antibody). Then, ELISA (enzyme-linked immunosorbent assay) at an extremely high sensitivity (described in Japanese Patent Laid-open No.57-152899)is employed for the assay of a trace substance, and non-specific adsorption causes a significant problem at separation.

### SUMMARY OF THE INVENTION

It is the objective of the present invention to remove contaminants; to shortening assay period; to simplify assay procedure; to eliminate non-specific binding; and to achieve quantitativeness, high sensitivity and low cost, and hence, to enable mass screening and automated analysis.

The present inventor has made various investigations in order to achieve the above objective. Consequently, he has found that by beforehand binding a polymer to an insoluble carrier, low molecular substances in a sample is adsorbed with no effect of the polymer, while an analytical subject polymer is not adsorbed. Thus, the present invention has been achieved.

The present invention relates to a method for separating an analytical subject polymer from a sample containing the polymer and a low molecular substance, comprising contacting to the sample an insoluble carrier bound with an identical polymer to or a different polymer from the analytical subject polymer to be substantially saturated, thereby adsorbing the low molecular substance onto the insoluble carrier, whereby the analytical subject polymer non-adsorbent to the carrier is separated from the sample, and also relates to a method for determining whether or not the low molecular substance is specifically bound to the analytical subject polymer, comprising contacting a mixture containing the analytical subject polymer and the low molecular substance to an insoluble carrier preliminarily bound with an identical polymer to or a different polymer from the analytical subject polymer to be substantially saturated, and analyzing the amount of the low molecular substance non-adsorbent to the insoluble carrier, thereby determining the amount of the low molecular substance specifically bound to the analytical subject polymer.

The present invention will now be explained in details hereinbelow.

The polymer to be bound to an insoluble carrier may be an oligomer or a higher polymer, specifically including natural polymers such as cellulose, carbohydrate, protein, nucleic acid, or natural polymers chemically modified therefrom; and further, including synthetic chemicals such as synthetic fiber and synthetic resin. From the viewpoint of cost, nucleic acids may preferably be those present at a vast amount, including those with less mechanical breaks during the purification stage thereof, for example, those derived from salmon- or herring sperm, and those from bacteria which can be cultured, phages or viruses or the like.

The polymerization degree of the polymer varies depending on the molecular weight and the partial specific volume of the low molecular substance in a sample to be adsorbed, and the degree is particularly under the effect of steric configuration. Thus, the limit is not clear. However, it is the minimum requirement that the size of the polymer molecule in its entirety is larger than the size of the entire low molecular weight molecule. That is, the polymerization degree of the polymer is two or more, preferably 5,000 or more, but varies depending on the type of the low molecular substance.

The method for binding the polymer to the insoluble carrier includes covalent bonds or physical adsorption due to non-covalent bonds, for example, hydrogen bonds, hydrophbic interaction, ionnic bonds, and van der Waals interaction. Any of these methods may be employed satisfactorily.

The insoluble carrier includes synthetic resins such as polystyrene, polyethylene, nitrocellulose, and nylon; natural products such as liposomes and phospholipid particles; adsorbent materials of inorganic materials such as glass, silicagel, magnetic substances, ceramics, celite, active alumina, and active charcoal, but the carrier is not limited to them. The insoluble carrier preferably contains at least these adsorbent materials.

Preferably, the low molecular substance in a sample has a lower molecular weight than the polymer to be bound to the insoluble carrier or a substance as the analytical subject. Also, the low molecular substance preferably is an organic substance. Specifically, the low molecular substance generally contains a labeled substance, but such low molecular substance satisfactorily may contain only a contaminant. The low molecular substance includes oligonucleotide, polynucleotide, and protein; the labeling substance for labeling includes radioactive isotopes, fluorescent substances, luminescent substances, dyes, enzymes, enzyme inhibitors, haptens, substrates or coenzymes capable of enzyme cycling reaction. More specifically, such oligonucleotide is illustrated by a probe and the like of a specific sequence of several tens bases, whereas such protein is illustrated by labeled antibodies, labeled antigens, labeled receptors, and enzymes. The fluorescent substance functioning as a labeling substance includes ethidium bromide, fluorescein, rhodamine, umbelliferon, and the like; the labeling enzyme includes triose phosphate isomerase, peroxidase, alkaline phosphatase, acid phosphatase, glucose oxidase, and β -galactosidase and the like.

The non-adsorbent polymer as the analytical subject substance in a sample may be either of an identical type to or a different type from the type of the polymer to be bound to the insoluble carrier, and the non-adsorbent polymer preferably has a higher molecular weight. Specifically, the polymer includes cellulose, carbohydrate, protein, nucleic acid, soluble synthetic fiber or synthetic resin. Herein, nucleic acid is specifically explained. Needless to say, other polymers are applied to this in the same way.

The nucleic acid in a sample is DNA or RNA, naturally occurring, synthesized or modified, and may be either of single strand or double strand. The base number of the nucleic acid is preferably 10,000 or more. Generally, the nucleic acid is analyzed by an appropriate detection system, using in many cases a labeled probe. Therefore, a target nucleotide sequence in a nucleic acid and a labeled probe are hybridized together at an appropriate temperature. The probe used for such detection is preferably the one labeled with biotin. The nucleic acid forming a hybrid together with a biotin-labeled probe further forms a complex with enzyme-labeled avidin. The complex cannot be adsorbed onto the insoluble carrier bound with the polymer, whereby the complex can be detected.

By binding nucleic acid directly or indirectly with an antigen or a ligand or the like, an antibody or a receptor can be assayed. In such case, a second labeled antibody is used as the detection system for sandwich assay. By using a similar system, the amount of an unlabeled antigen can be assayed by competition between nucleic acid bound with an antigen and the labeled antibody. This is the case with ligand. Representative examples of an antigen or ligand to be assayed include thyrotropin, thyroxine, growth hormone, insulin, placental gonadotropin, steroid hormone, drugs and the like.

The enzyme employing nucleic acid as a substrate or a template includes DNA polymerase, RNA polymerase, terminal transferase, polynucleotide kinase, nuclease S1, ligase or restriction enzyme. Because these enzymes and DNA binding proteins can be bound with nucleic acid, they can be assayed as described above. In order to assay the activity of terminal transferase, for example, naturally occurring DNA is used as a substrate for binding, via enzyme action, deoxynucleotide 3-phosphate bound with biotin at 3' terminus, and the bound biotin is then assayed.

Centrifuge, column or filter or the like is used as the separation means between a labeled substance bound to the non-adsorbent polymer as an analytical subject and an insoluble carrier.

In case that a sample is a mixture containing a low molecular substance and a labeled polymer as an analytical substance, the low molecular substance is adsorbed onto the insoluble carrier if the sample is added to the carrier bound with the polymer to be substantially saturated. Then, only the labeled polymer is separated. Subsequently, the separated polymer is subjected to analysis with an appropriate detection system.

The present invention is an excellent method, extremely simplified, because a sample can be prepared in solution, and because a sample can be analyzed for a very short time by charging the sample on a column packed with the insoluble carrier bound with the polymer and subsequently eluting the sample, which achieve the measurement of a great number of samples, thereby enabling the application of the method to mass screening and automatic analysis which have conventionally been difficult.

The most characteristic feature of the present invention is such that the insoluble carrier bound with the polymer (specifically DNA-bound active charcoal) specifically adsorbs a non-bound labeled low molecular substance and a contaminant in a sample containing the low molecular substance and the contaminant which interferes with a labeled analytical subject during detection, thereby reducing background and achieving highly sensitive assay. In particular, the labeled low-molecular substance in accordance with the present invention includes a labeled enzyme having a molecular weight of around several hundreds of thousands.

The insoluble carrier to be used in accordance with the present invention is extremely inexpensive in terms of cost, because an adsorbent of an inorganic material can be utilized therein.

The present invention has advantages thus described, so it can be utilized as extremely innovative enzyme assay, enzyme immunoassay, and gene analysis.

### DESCRIPTION OF PREFERRED EMBODIMENTS

### Example 1

### 1. Preparation of DNA-bound carrier

Two-hundred milliliters of 1 mg/ml salmon DNA were added to 100 g of active charcoal for chromatography (WAKO Chemicals, Co. Ltd.), which was then left to stand overnight at room temperature followed by washing in a solution of 0.5 M ammonium sulfate, 1 mM EDTA, pH 7.2. The active charcoal thus treated was charged onto a column to a bed volume of 2 ml.

### 2. Probe for detecting point mutation

The following seven types of probes were purchased from TAKARA LIQUOR, CO. LTD.; the first probe is a naturally occurring type, and the second to seventh types of the probes have a point mutation at the codon of 12-th amino acid residue of c-Ki-ras gene.

5'-GTTGGAGCTGGTGGCGTAGG-3' (Gly)
5'-GTTGGAGCTCGTGGCGTAGG-3' (Arg)
5'-GTTGGAGCTTGTGGCGTAGG-3' (Cys)
5'-GTTGGAGCTAGTGGCGTAGG-3' (Ser)
5'-GTTGGAGCTGCTGGCGTAGG-3' (Ala)
5'-GTTGGAGCTGATGGCGTAGG-3' (Asp)
5'-GTTGGAGCTGTTGGCGTAGG-3' (Val)

### 3. Preparation of probe with a biotinylated terminus

The terminus of each of the probes described above was biotinylated with final concentrations of 0.2 M potassium cacodylate, pH 6.6, 10 µM each probe, 2.5 mM cobalt chloride, 500 U/ml terminal transferase, and 30 µM biotin-16-dUTP under reaction conditions of 37 ^{.}C for 10 hours.

### 4. Hybridization and detection

A mixture solution composed of 0.5 mg/ml salmon DNA, 80 mM sodium chloride, 20 mM sodium phosphate, pH7.0, 10 nM probe with a biotinylated terminus was charged into an Eppendorf tube, and heated in boiling water for 5 minutes followed by standing alone at room temperature. After 5 minutes at room temperature, peroxidase-bound streptoavidin (Amersham) was added to a final 1000-fold dilution. Thirty minutes later, 100 µl of a sample was added onto an active charcoal column, followed by elution with 2 ml of a solution of 0.5 M ammonium sulfate, 1 mM EDTA, pH 7.2. The time period required for such elution is within 5 minutes. A color reagent (2 ml; 2 mM TMBZ, 10 mM hydrogen peroxide, citric acid/citrate buffer, pH 4.6) was further added to the eluent. Twenty minutes later, absorbance of the solution was measured at 650 nm with a colorimeter. Thus, the results shown in the following Table 1 were obtained.

**Table 1**

| Probe | Absorbance at 650 nm |
|---|---|
| probe (Gly) | 0.148 |
| probe (Arg) | 0.066 |
| probe (Cys) | 0.068 |
| probe (Ser) | 0.072 |
| probe (Ala) | 0.048 |
| probe (Asp) | 0.076 |
| probe (Val) | 0.060 |
| no probe | 0.052 |

As is clearly shown in the results, the probe having the sequence of the naturally occurring type hybridizes with salmon DNA, with no adsorption onto the column, and has a distinctively higher absorbance than other probes. In accordance with the method of the present invention, point mutation in genomic DNA can be assayed with an extremely high specificity at a higher sensitivity. The procedures thereof are very simple and can achieve the automated analysis.

### Example 2

A mixture solution composed of 0.5 mg/ml herring DNA, 80 mM sodium chloride, 20 mM sodium phosphate, pH7.0, 10 nM oligodeoxycytidylic acid (polymerized number of 24 to 36) with a biotinylated terminus was charged into an Eppendorf tube and heated in boiling water for 15 minutes, followed by standing alone at room temperature. After 20 minutes at room temperature, peroxidase-bound streptoavidin (Amersham) was added to final dilutions of 1,000-fold, 2,000-fold, 4,000-fold and 8,000-fold. Twenty minutes later, 100 µl of a sample was further added onto an active charcoal column, followed by elution with 2 ml of a solution of 0.5 M ammonium sulfate, 1 mM EDTA, pH 7.2. A color reagent (2 ml; 2 mM TMBZ, 10 mM hydrogen peroxide, citric acid/citrate buffer, pH 4.6) was added to the solution. Fifteen minutes later, absorbance of the solution was measured at 650 nm with a colorimeter. Thus, the results shown in the following Table 2 were obtained.

**Table 2**

| Condition | Absorbance at 650 nm |
|---|---|
| 8000-fold dilution | 0.020 |
| 4000-fold dilution | 0.040 |
| 2000-fold dilution | 0.064 |
| 1000-fold dilution | 0.168 |

The results show the proportionality with the enzyme concentration, which indicate good quantitativeness of the method of the present invention. In the present example, biotin is used instead of ligand or hapten, while peroxidase-bound streptoavidin is used as a representative example of a receptor or an antibody. Therefore, the present invention can absolutely be applied to a wide range of fields, for example, proteins capable of binding to nucleic acid, enzymes required for nucleic acid analysis, and enzyme analysis for blood coagulation, as well as antigen-antibody reaction, and binding of a ligand and a receptor.

### Example 3

### 1. Preparation of DNA-bound carrier

Two-hundred milliliters of 1 mg/ml salmon DNA, denatured by heating, were added to 100 g of active charcoal for chromatography (WAKO Chemicals, Co. Ltd.), which was then left to stand at room temperature overnight. The resulting mixture was washed in a solution of 0.5 M ammonium sulfate, 1 mM EDTA, pH 7.2. The active charcoal thus treated was charged onto a column to a bed volume of 1 ml.

### 2. Probe for detecting point mutation

By using a DNA synthesizer manufactured by Millipore, Co. Ltd, the following seven types of probes each having biotinylated 5' terminus were synthesized by phosphoamidite method. These have a point mutation at the codon of 12-th amino acid residue of c-Ki-ras gene.

5'-Biotin-GTTGGAGCTGGTGGCGTAGG-3' (Gly)
5'-Biotin-GTTGGAGCTCGTGGCGTAGG-3' (Arg)
5'-Biotin-GTTGGAGCTTGTGGCGTAGG-3' (Cys)
5'-Biotin-GTTGGAGCTAGTGGCGTAGG-3' (Ser)
5'-Biotin-GTTGGAGCTGCTGGCGTAGG-3' (Ala)
5'-Biotin-GTTGGAGCTGATGGCGTAGG-3' (Asp)
5'-Biotin-GTTGGAGCTGTTGGCGTAGG-3' (Val)

### 3. Hybridization and detection

A mixture solution of 200 µg/ml human DNA, 250 mM ammonium sulfate, 1 mM EDTA, pH 7.0, and 20 nM probe with a biotinylated terminus was charged into an Eppendorf tube and heated in boiling water for 5 minutes followed by standing alone at room temperature. After 5 minutes at room temperature, peroxidase-bound streptoavidin (Amersham) was added to a final 500-fold dilution. Ten minutes later, 30 µl of a sample was added to an active charcoal column followed by elution with 2 ml of a solution of 0.5 M ammonium sulfate, 1 mM EDTA, pH 7.2. The time period required for such elution is within 15 minutes. A color reagent (100 µl; 10 mM TMBZ, 10 mM hydrogen peroxide, citric acid/citrate buffer, pH 4.6) was added to the eluent. Twenty minutes later, absorbance of the solution was measured at 650 nm with a colorimeter. Thus, the results shown in the following Table 3 were obtained.

**Table 3**

| Probe | Absorbance at 650 nm |
|---|---|
| probe (Gly) | 0.155 |
| probe (Arg) | 0.032 |
| probe (Cys) | 0.040 |
| probe (Ser) | 0.024 |
| probe (Ala) | 0.045 |
| probe (Asp) | 0.034 |
| probe (Val) | 0.050 |
| no probe | 0.017 |

### Example 4

The same experiment as in Example 3 was performed, provided that the following normal type probe of c-Ki-ras/61 was employed;
5'-Biotin-ACAGCAGGTCAAGAGGAGTA-3'.
Active alumina was used in place of active charcoal; in place of peroxidase-bound streptoavidin, peroxidase-labeled biotin monoclonal antibody, Fab fragment (Behringer Mannheim) was used.

The results indicate that, the absorbance at 650 nm was 0.356 when the probe was bound to DNA and that of the control without probe was 0.057. The data shows that the biotin bound to the probe can function in place of hapten in antigen-antibody reaction. Therefore, hapten or antibody as an analytical subject can be assayed by the method.

## Claims

1. A method for separating an analytical subject polymer from a sample containing the polymer and a low molecular substance, comprising contacting to the sample an insoluble carrier bound with an identical polymer to or a different polymer from the analytical subject polymer to be substantially saturated, thereby adsorbing the low molecular substance onto the insoluble carrier, whereby the analytical subject polymer non-adsorbent to the insoluble carrier is separated from the sample.

2. A method for determining whether or not a low molecular substance is specifically bound to an analytical subject polymer, comprising contacting a mixture containing the analytical subject polymer and a low molecular substance to an insoluble carrier preliminarily bound with an identical polymer to or a different polymer from the analytical subject polymer to be substantially saturated, and analyzing the amount of the low molecular substance non-adsorbent to the insoluble carrier, thereby determining the amount of the low molecular substance specifically bound to the analytical subject polymer,

3. The method according to claim 2, wherein the polymer bound to the insoluble carrier is a polymer from a living organism or a synthesized chemical.

4. The method according to claim 3, wherein the polymer from a living organism is nucleic acid.

5. The method according to claim 4, wherein the nucleic acid is DNA.

6. The method according to claim 2, wherein the insoluble carrier is an adsorbent.

7. The method according to claim 6, wherein the adsorbent includes active alumina or active charcoal.

8. The method according to claim 2, wherein the low molecular substance has a lower molecular weight than the weight of the analytical subject polymer.

9. The method according to claim 2 or 8, wherein the low molecular substance contains at least one of oligonucleotide, polynucleotide, and protein.

10. The method according to claim 9, wherein the oligonucleotide or polynucleotide is a labeled probe to be used for hybridization.

11. The method according to claim 9, wherein the protein is a labeled antibody.

12. The method according to claim 2 or 11, wherein the analytical subject polymer is nucleic acid.

13. The method according to claim 12, wherein the base number in the nucleic acid is 10,000 bases or more.

14. The method according to claim 12 or 13, wherein the nucleic acid is the one binding an antigen or a ligand.

15. A method for mesuring the amount of antigen comprising contacting a mixture including the antigen, nucleic acid bound with the same antigen and labeled antibody with an insoluble carrier bound with polymer to be substantially saturated, and analyzing the amount of the labeled antibody non-adsorbent to the insoluble carrier.

16. The method according to claim 2, wherein the analytical subject polymer is nucleic acid and the low molecular substance is an oligonucleotide probe to the nucleic acid.

17. The method according to claim 16, comprising binding the antigen bound to a probe with a labeled antibody to the antigen, measuring the amount of the labeled antibody non-adsorbent to the insoluble carrier, thereby determining the amount of the probe bound to the nucleic acid.

18. The method according to claim 17, wherein the antigen is hapten.

19. The method according to claim 17, wherein the labeled antibody is the one directly or indirectly labeled with either one of enzyme, fluorescent substance, luminescent substance, dye, coenzyme, hapten, radioactive isotope, or a substrate capable of enzyme cycling.

20. The method according to claim 19, wherein the enzyme is selected from triose phosphate isomerase, peroxidase, alkaline phosphatase, acid phosphatase, and β-galactosidase.

21. The method according to claim 12, wherein the analytical subject nucleic acid functions as a substrate or a template for enzyme reaction.

22. The method according to claim 21, wherein the enzyme is selected from DNA polymerase, RNA polymerase, terminal transferase, polynucleotide kinase, nuclease S1, and ligase.
